(19) Europäisches Patentamt
European Patent Office
Office européen des brevets

(11) Publication number: **0 084 974**
**B1**

(12) **EUROPEAN PATENT SPECIFICATION**

(45) Date of publication of patent specification: **02.04.86**

(21) Application number: **83300354.4**

(22) Date of filing: **24.01.83**

(51) Int. Cl.$^4$: **C 07 C 13/28,** C 07 C 43/21, C 07 C 43/215, C 07 C 43/184, C 09 K 19/30 // C07C35/21, C07C25/18, C07F3/02, C07C49/613, C07C69/157, C07C43/23

# (54) Carbocyclic compounds with liquid-crystal properties.

(30) Priority: **22.01.82 JP 8291/82**
**16.02.82 JP 23438/82**
**08.03.82 JP 36100/82**
**01.04.82 JP 54080/82**
**01.04.82 JP 54081/82**
**10.05.82 JP 77928/82**
**20.05.82 JP 85272/82**
**02.07.82 JP 115181/82**
**02.07.82 JP 115182/82**

(43) Date of publication of application:
**03.08.83 Bulletin 83/31**

(45) Publication of the grant of the patent:
**02.04.86 Bulletin 86/14**

(84) Designated Contracting States:
**CH DE GB LI**

(58) References cited:
**EP-A-0 030 277**
**EP-A-0 048 141**
**US-A-2 233 964**
**US-A-2 263 448**

(73) Proprietor: **Chisso Corporation**
**6-32, Nakanoshima 3-chome Kita-ku**
**Osaka-shi Osaka-fu (JP)**

(72) Inventor: **Sugimori, Shigeru**
**2493-10. Fujisawa**
**Fujisawa-shi Kanagawa-ken (JP)**
Inventor: **Kojima, Tetsuhiko**
**10-3, Otsutomo-cho Kanazawa-ku**
**Yokohama-shi Kanagawa-ken (JP)**

(74) Representative: **Ruffles, Graham Keith et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 084 974 B1**

**Description**

This invention relates to new carbocylic compounds having 4 or 5 six-membered rings which exhibit a liquid-crystalline phase over a broad temperature range, and compositions containing the same.

Display elements using liquid crystals have come to be widely used, for example in watches and calculators. Such display elements utilize optical anisotropy and dielectric anisotropy of liquid-crystalline substances in the nematic, smectic or cholesteric phase, although those utilizing nematic liquid crystals have been most widely employed in practice. Liquid-crystalline display elements include those of the TN type (twisted nematic type), DS type (dynamic scattering type), guest-host type and DAP type, and require liquid-crystalline substances of varying properties; but those which exhibit a liquid-crystalline phase within as broad a range as possible of ambient conditions are desirable. However, no single compounds satisfying such a requirement have been found to date, so that liquid-crystal compositions which are practically usable for the present have been obtained by mixing together several different kinds of liquid-crystalline or non-liquid-crystalline compounds.

These substances must, of course, be stable to moisture, heat and air. Moreover, the threshold voltage and saturation voltage required for driving the display elements should be as low as possible. The viscosity should also be as low as possible, to make the response speed higher; but, in order to broaden the liquid-crystalline temperature range toward the upper end, it is necessary to employ a high melting liquid-crystalline substance as a component, and such a substance generally has a high viscosity, so that a liquid-crystalline composition containing it also has a high viscosity. Hence, there has been a tendency for liquid-crystalline display elements usable up to, eg, 80°C to have a markedly slow response speed, particularly at lower temperatures.

In our laid-open Japanese patent applications No 165,328/1982 corresponding to EP—A—62 470, we have disclosed 1-alkyl-4-[trans-4-(trans-4-alkylcyclohexyl)cyclohexyl] benzenes of the general formula

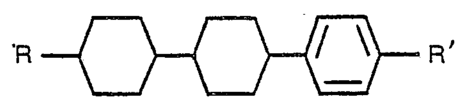

as liquid-crystalline compounds with improved low temperature characteristics. However, recent advances in liquid-crystalline display elements now call for a broader useable temperature range. The present invention provides liquid-crystalline compounds meeting these requirements, that is exhibiting a liquid-crystalline phase within a broader temperature range and also have a high transparent point and yet a low viscosity.

EP—A—30 277 describes liquid-crystalline compounds of the formula (I)

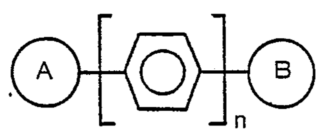

wherein *n* is 1 or 2 and the rings A and B are the same or different and are trans-4-alkylcyclohexyl or 4-alkylcyclohexyl-1-yl.

The present invention provides liquid-crystalline compounds with 4 or 5 six-membered rings. The compounds are of the following general formulae (II) to (X):

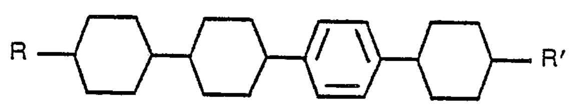

(II)

wherein each of R and R′ represents a hydrogen atom or an alkyl group of 1 to 10 carbon atoms;

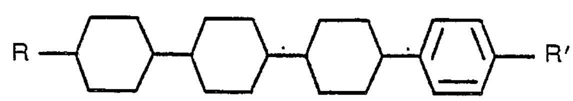

(III)

wherein each of R and R′ represents a hydrogen atom, or an alkyl or alkoxy group, each of 1 to 10 carbon atoms;

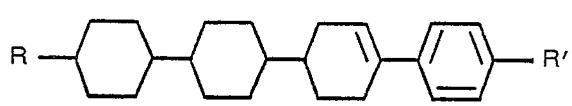

(IV)

wherein each of R and R′ represents a hydrogen atom, or an alkyl or alkoxy group, each of 1 to 10 carbon atoms;

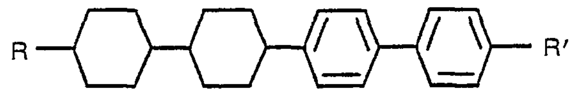

(V)

wherein each of R and R′ represents a hydrogen atom, or an alkyl or alkoxy group, each of 1 to 10 carbon atoms;

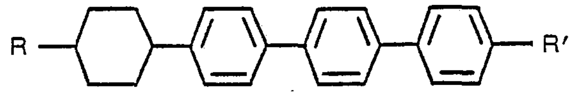

(VI)

wherein each of R and R′ represents a hydrogen atom, or an alkyl or alkoxy group, each of 1 to 10 carbon atoms;

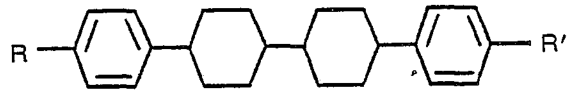

(VII)

wherein R represents a hydrogen atom, or an alkyl or alkoxy group, each of 1 to 10 carbon atoms;

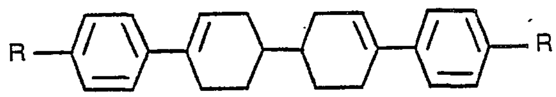

(VIII)

wherein R represents a hydrogen atom, or an alkyl or alkoxy group, each of 1 to 10 carbon atoms;

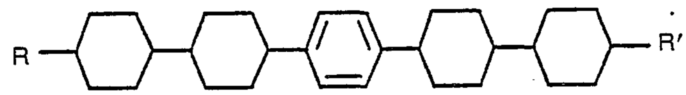

(IX)

wherein each of R and R′ represents a hydrogen atom, or an alkyl group of 1 to 10 carbon atoms;

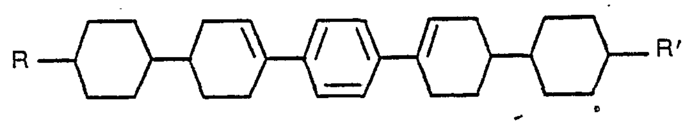

(X)

wherein each of R and R′ represents a hydrogen atom, or an alkyl group of 1 to 10 carbon atoms.

In the above formulae (II) to (X), when R and R′ are both present, they may be the same or different from each other.

The invention also provides compositions containing at least of one of said compounds of formula (II) to (X).

The compounds of the present invention have a broad liquid-crystalline temperature range extending to relatively high temperatures, such that the liquid-crystalline-transparent points are in the vicinity of 300°C, and nevertheless have a relatively low viscosity. When they are mixed in a small amount with other liquid-crystalline compounds such as esters, biphenyl, phenyl-cyclohexane, Schiff or apoxy liquid-crystalline compounds, or hetero-ring-containing liquid-crystalline compounds, they are very useful in cells for liquid crystal display elements capable of being actuated over a broad temperature range. Their high liquid-crystalline-transparent point enables them to be used in a small amount in such compositions, without greatly raising the threshold voltage and the saturation voltage of the resulting liquid-crystalline compounds. The compounds of the present invention are also stable to light, heat, air and moisture, and have a very broad range of applications.

Preparation of compounds of the formula (II)

p-Dibromobenzene and metallic magnesium are reacted to give 4-bromobenzenemagnesium bromide, which is then reacted with a 4-alkylcyclohexanone to obtain a 4-(4′-alkylcyclo-hexan-1-ol)bromobenzene. This is then dehydrated in the presence of potassium hydrogen sulphate as catalyst, to obtain a 4-(4′-alkylcyclohexen-1-yl)bromobenzene, which is then converted with metallic magnesium into a 4-(4′-alkylcyclohexen-1-yl)benzenemagnesium bromide. This, in turn, is then subjected to catalytic reduction in an autoclave, in the presence of Raney Ni as catalyst, followed by recrystallization, to obtain the desired product, a 1-(trans-4-alkylcyclohexyl)-4-[trans-4-(trans-4-alkylcyclohexyl)cyclohexyl]benzene (II). The above steps are expressed by the following equations:

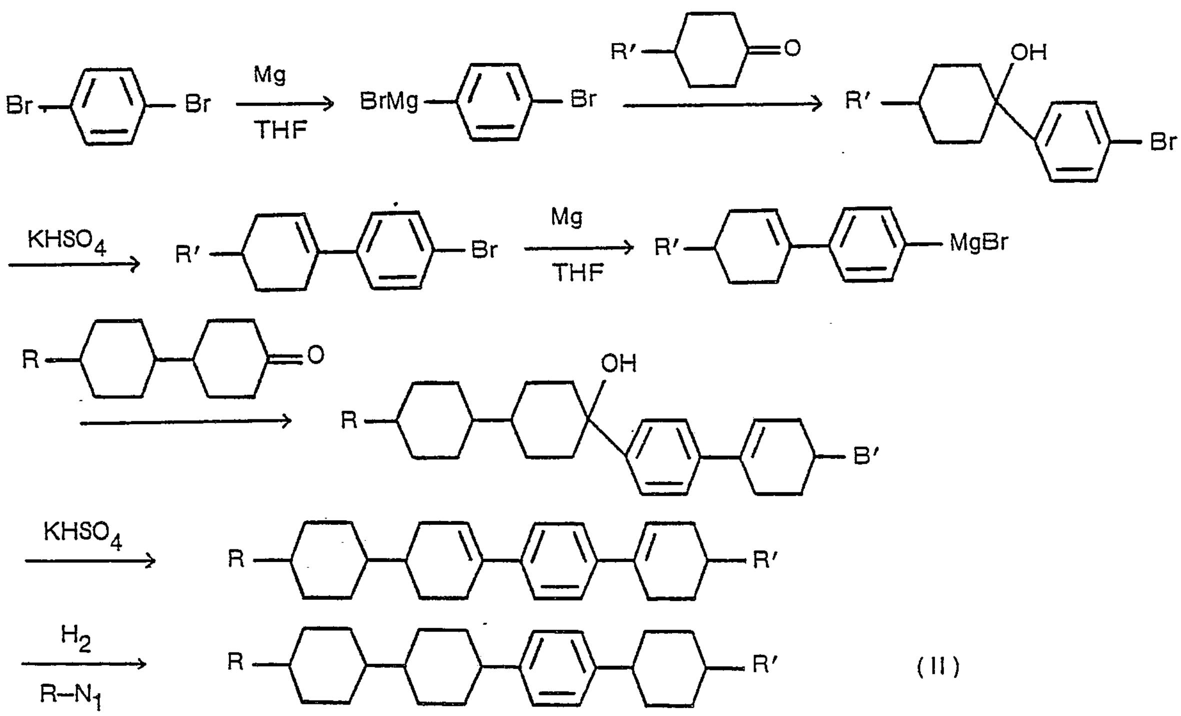


(II)

Preparation of compounds of the formulae (V) and (VI)

Metallic Mg is reacted with a 4-substituted-4′-bromo-biphenyl, to obtain a 4-substituted-4′-biphenylmagnesium bromide, which is then reacted with a 4-(trans-4-substituted-cyclohexyl)cyclohexanone, to obtain a 4-substituted-4′-[4-(trans-4-substituted-cyclohexyl)-cyclohexan-1-ol-]biphenyl. This is then dehydrated in the presence of potassium hydrogen sulphate as catalyst to obtain a 4-substituted-4′-[4-(trans-4-substituted-cyclohexyl)-cyclohexen-1-yl]biphenyl (XI), which is reduced under atomspheric pressure at 50°C using Raney Ni as catalyst is n-heptane. The cis- and trans-isomers of the product are separated by recrystallization, to obtain the desired product, a 4-substituted-4′-[4-trans-4-(trans-4-substituted-cyclohexyl)cyclohexyl]biphenyl (V).

The above steps are expressed by the following equations:-

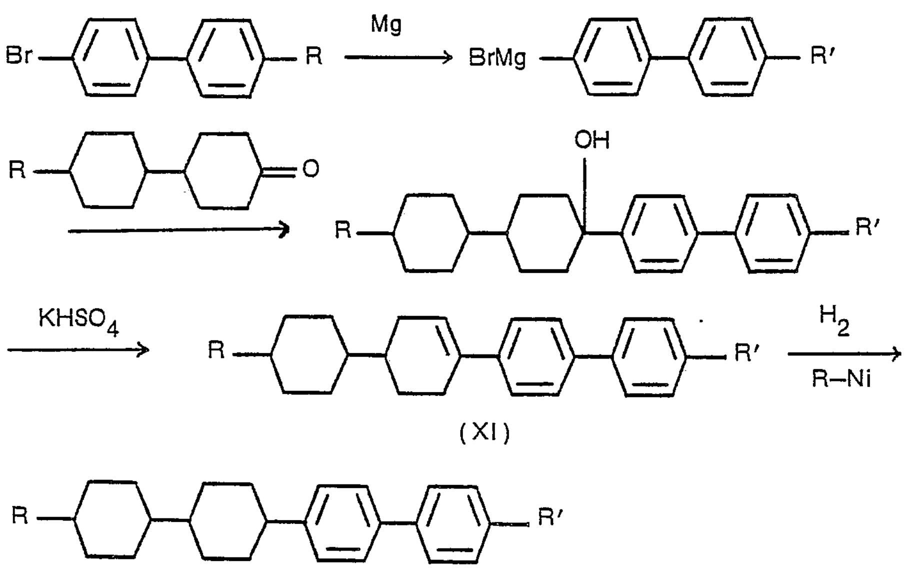


(XI)

(V)

The above compounds (V) can also be obtained according to the following method:

Metallic Mg is reacted with 4-bromobiphenyl, to obtain 4-biphenylmagnesium bromide, which is then reacted with a 4-(trans-4-substituted-cyclohexyl)-cyclohexanone to obtain a 4-[4-(trans-4-substituted-cyclohexyl)cyclohexan-1-ol]-biphenyl. This is then dehydrated with potassium hydrogen sulphate, to obtain

a 4-[4-trans-4-substituted-cyclohexyl)cyclohexen-1-yl]biphenyl, which is then reduced with Raney Ni catalyst to obtain a 4-[trans-4-(trans-4-substituted-cyclohexyl)cyclohexyl]biphenyl. This, in turn, is subjected to a Friedel-Krafts reaction with aluminium chloride and an alkanoyl chloride, to obtain a 4-alkanoyl-4'-[trans-4-(trans-4-substituted-cyclohexyl)-cyclohexyl]biphenyl, which is then subjected to a Wolff-Kishner reduction to obtain the desired product (V).

These steps are shown in the following reaction schemes:-

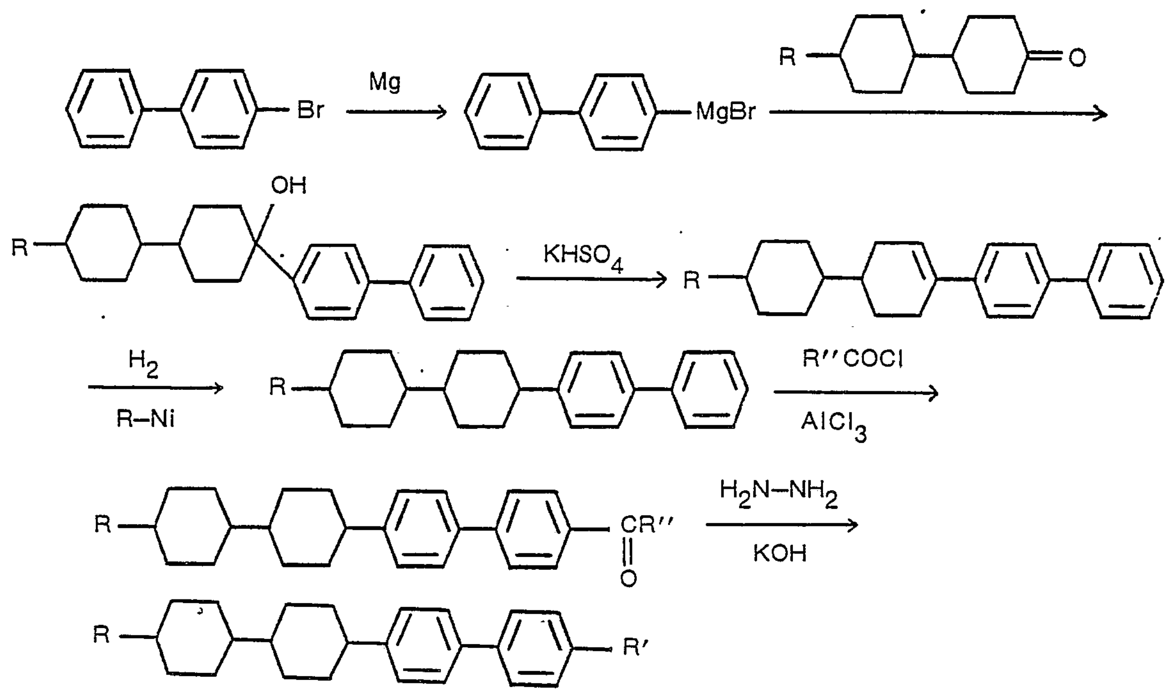


(V)

Preparation of compounds of formula (VI)

A compound of the formula (XI), obtained in the above preparation of the compounds of the formula (V), is dehydrogenated with chloranil to obtain the desired product, a 4-substituted-4'-(trans-4-alkylcyclohexyl)terphenyl.

The above steps are expressed by the following chemical equation:-

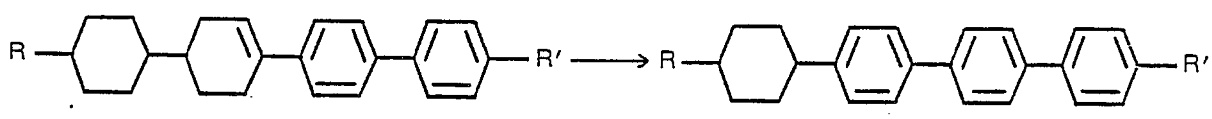

(XI) (VI)

Preparation of compounds of formula (III) and (IV)

Metallic Mg is reacted with 4-methoxybromobenzene to obtain 4-methoxybenzenemagnesium bromide, which is then reacted with a 4-(trans-4-alkylcyclohexyl)cyclohexanone to obtain a 4-methoxy-[4-trans-4-alkylcyclohexyl)cyclohexen-1-ol]benzene. This is dehydrated to obtain a 4-methoxy[4-trans-4-alkylcyclohexyl)cyclohexen-1-yl]-benzene, which is then reduced with Raney Ni catalyst, followed by recrystallization, to obtain a 4-methoxy-[4-(trans-4-alkylcyclohexyl)cyclohexyl]benzene. This is then subjected to demethylation with hydrobromic acid to obtain a 4-hydroxy-[trans-4-(trans-4-alkylcyclohexyl)cyclohexyl]-benzene, which is then subjected to catalytic reduction under 50 $kg/cm^2$ at 150°C with Ru/C catalyst to obtain a 4-[trans-4-(trans-4-alkylcyclohexyl)cyclohexyl]cyclohexanol, which is in turn subjected to oxidation with chromic acidsulphuric acid to obtain a 4-[trans-4-(trans-4-alkylcyclohexyl)cyclohexyl]cyclohexanone(XII). Compound (XII) is added dropwise to a Grignard reagent, obtained by reacting a 4-substituted-bromobenzene with Mg, to give a 4-substituted-1-{4-[trans-4-(trans-4-alkylcyclohexyl)-cyclohexyl]cyclohexan-1-ol}benzene, which is then dehydrated to obtain a 4-substituted-{4-[trans-4-(trans-4-alkylcyclohexyl)cyclohexyl]cyclohexen-1-yl}benzene (IV). Lastly, compound IV) is reduced with Raney Ni catalyst, followed by recrystallization to obtain the desired product, a 4-substituted-{4trans-4-[trans-4-(trans-4-alkylcyclohexyl)-cyclohexyl]cyclohexyl}benzene (III).

The above steps are expressed by the following chemical equations:-

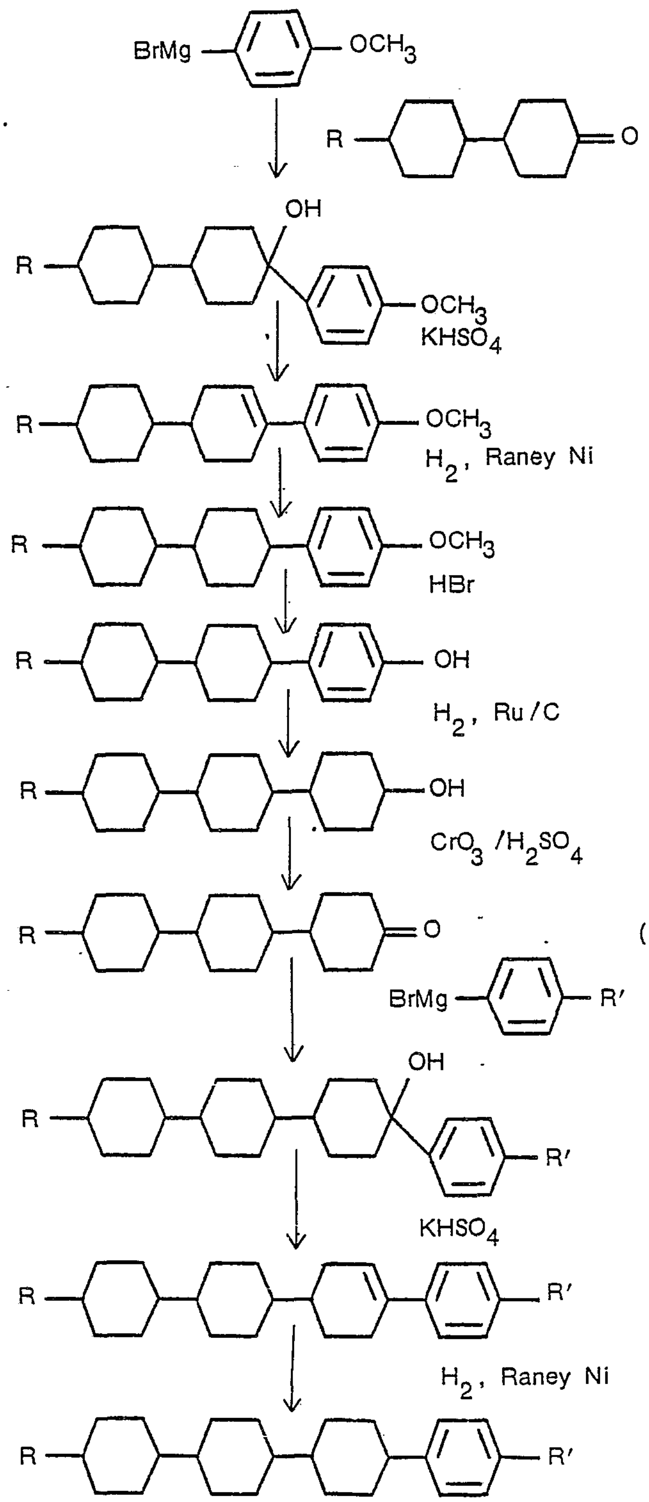


(XII)

(IV)

(III)

Preparation of compounds of formulae (VII) and (VIII)

Metallic magnesium is reacted with a 4-substituted-bromobenzene to obtain a 4-substituted-phenylmagnesium bromide, which is then reacted with 4,4′-bicyclohexanedione to obtain a 4,4′-bis(4-substituted-phenyl)bicyclohexan-4,4′-diol. This is then dehydrated with potassium hydrogen sulphate catalyst, to obtain a 4,4′-bis(4-substituted-phenyl)bicyclohexan-3,3′-diene (VIII), which is then subjected to catalytic reduction in an autoclave with Raney Ni catalyst, followed by recrystallization, to obtain the desired product, a trans,trans-4,4′-(4-substituted-phenyl)bicyclohexane (VII).

The above steps are expressed by the following chemical equations:

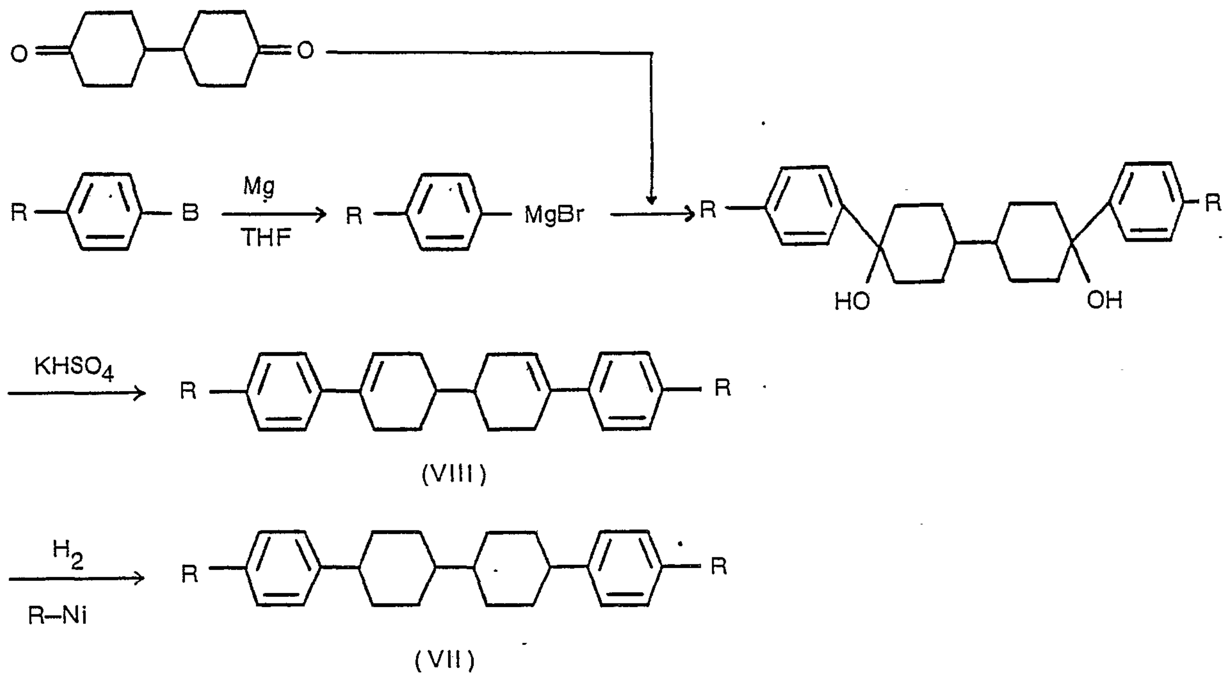


(VIII)

(VII)

Preparation of compounds of formulae (IX) and (X)

Metallic magnesium is reacted with p-dibromobenzene, to obtain p-phenylmagnesium bromide, which is then reacted to a 4-(trans-4-substituted-cyclohexyl)cyclohexanone, to give a 1,4-bis[4-(trans-4-alkylcyclohexyl)cyclohexan-1-ol]benzene. This is dehydrated with potassium hydrogen sulphate catalyst, to obtain a 1,4-bis[4-(trans-4-alkylcyclohexyl)cyclohexen-1-yl]benzene (X), which is then reduced with Raney Ni to obtain the desired product, a 1,4-bis[trans-4-(trans-4-alkylcyclohexyl)]benzene (IX).

The above steps are expressed by the following chemical equations:

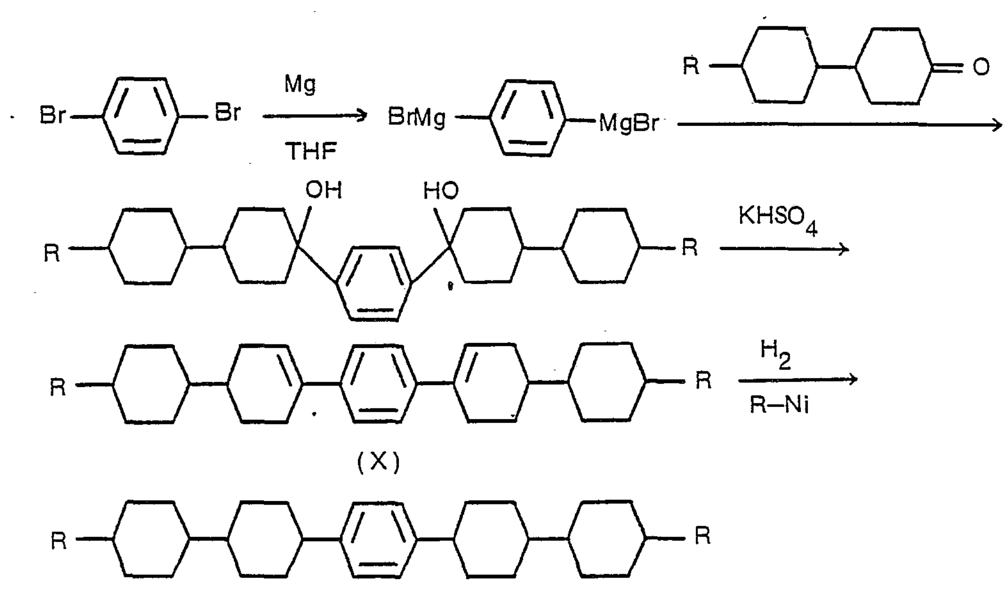


(X)

(IX)

The invention is illustrated by the following Examples.

EXAMPLE 1

Preparation of 1-(trans-4-methylcyclohexyl)-4-[trans-4-(trans-4-pentylcyclohexyl)cyclohexyl]benzene (a compound of the general formula (II) wherein R = $C_5H_{11}$ and R′ = $CH_3$).

Sliced magnesium (29.2 g, 1.20 mol) was placed in a three-neck flask, and a solution obtained by dissolving p-dibromobenzene (283.1 g, 1.20 mol) in tetrahydrofuran (500 ml) was slowly added dropwise to the magnesium under a nitrogen current with stirring while the reaction temperature was kept at 30 to 35°C.

After 3 hours, when the reaction to form the magnesium bromide was complete, a solution obtained by dissolving 4-methylcyclohexanone (107.1 g, 1.00 mol) in tetrahydrofuran (200 ml) was rapidly added dropwise while the reaction temperature was kept below 30°C. After the addition, the mixture was refluxed for 20 minutes and then acidified with 3N hydrochloric acid. The reaction liquid was extracted with n-heptane (three times 300 ml). The n-heptane layers were combined and washed with water, followed by distilling off the solvent under reduced pressure.

The residual oily substance was 4-(4-methylcyclohexan-1-ol)bromobenzene, to which potassium hydrogen sulphate (30 g) was added, followed by dehydration at 200°C for 2 hours under a nitrogen current. After cooling, n-heptane (1 litre) was added and the potassium hydrogen sulphate was filtered off, followed by washing the n-heptane layer with water and distilling off the solvent under reduced pressure. The residue was distilled under reduced pressure and the main fractions (170 to 173°C/9 mmHg) were collected, followed by recrystallization from ethanol, to obtain 4-(4-methylcyclohexen-1-yl)bromobenzene.

This bromobenzene (6.1 g, 0.024 mol) was dissolved in tetrahydrofuran (20 ml) and reacted with metallic magnesium (0.58 g, 0.024 mol) under a nitrogen current to obtain 4-(4-methylcyclohexen-1-yl)benzenemagnesium bromide.

To this was added a solution obtained by dissolving 4-(trans-4-pentylcyclohexyl)cyclohexanone (4.8 g, 0.019 mol) in tetrahydrofuran (20 ml), and the resulting mixture was extracted with n-heptane (300 ml). The oily layer was washed with water and the solvent was distilled off under reduced pressure. Potassium hydrogen sulphate (2 g) was added to the residue, followed by dehydration at 200°C for 2 hours under a nitrogen current. After cooling, toluene (200 ml) was added, the potassium hydrogen sulphate was filtered off and the toluene layer was washed with water. The solvent was distilled off under reduced pressure and the residue was recrystallized from toluene, to obtain 1-(4′-methylcyclohexen-1-yl)-4-[-4-(trans-4-pentylcyclohexyl)cyclohexen-1-yl]benzene.

This material (1.2 g) was dissolved in toluene (20 ml) and subjected to catalytic hydrogenation in an autoclave with Raney Ni (1.0 g). The reaction was followed by gas chromatography and, when the raw material had disappeared, the catalyst was filtered off and the desired product, 1-(trans-4-methylcyclohexyl)-4-[trans-4-(trans-4-pentylcyclohexyl)clyclohexyl]benzene was isolated by repeated recrystallisation (yield: 0.1 g). This product had a crystalline-smectic point (C-Sm point) of 30°C or lower, a smectic-nematic point (Sm-N point) of 256°C and a nematic-transparent point (N-I point) of 259°C.

EXAMPLES 2—5

Various compounds having different substituents were prepared in an analogous manner to that of Example 1. Their properties are shown in Table 1, together with those of the product of Example 1. Their chemical structures were confirmed by NMR.

TABLE 1

| Example | In formula (II) | | Yield (g) | Phase transition point (°C) | | |
|---|---|---|---|---|---|---|
| | R | R′ | | C—Sm point | Sm—N point | N—I point |
| 2 | $C_3H_7$ | $CH_3$ | 0.1 | 30 or lower | — | 300 or higher (Sm—I) |
| 3 | $C_3H_7$ | $C_2H_5$ | 0.2 | 30 or lower | 274 | 287 |
| 4 | $C_3H_7$ | $C_3H_7$ | 0.5 | 55 | 264 | 300 or higher |
| 1 | $C_5H_{11}$ | $CH_3$ | 0.1 | 30 or lower | 256 | 259 |
| 5 | $C_5H_{11}$ | $C_6H_{13}$ | 0.1 | 49 | — | 286 (Sm—I) |

EXAMPLE 6

Preparation of 4-pentyl-4′-[trans-4-(trans-4-pentylcyclohexyl)cyclohexyl]biphenyl (a compound of the formula (V) wherein R = R′ = $C_5H_{11}$).

A solution of 4-pentyl-4′-bromobiphenyl (2.8 g, 0.0092 mol) dissolved in tetrahydrofuran (50 ml) was

reacted with Mg (0.22 g, 0.0092 mol) under a nitrogen current, to obtain 4-pentyl-4'-biphenylmagnesium bromide. The reaction liquid was cooled to 0°C and to it was added a tetrahydrofuran solution (50 ml) of 4-pentylcyclohexyl-cyclohexanone (2.3 g, 0.0092 mol). The mixture was refluxed at 50°C for 2 hours and then acidified with 3N hydrochloric acid (50 ml), followed by extraction with toluene (200 ml), washing the extract with water, distilling off the solvent, adding potassium hydrogen sulphate (4 g) and dehydrating at 200°C for 2 hours under a nitrogen atmosphere. After cooling, toluene (200 ml) was added and the potassium hydrogen sulphate was filtered off, followed by washing with water till the washing water became neutral, dehydrating with anhydrous sodium sulphate, distilling off the solvent and recrystallizing from toluene to obtain crystals of 4-pentyl-4'-[-4-(trans-4-pentylcyclohexyl)cyclohexen-1-yl]biphenyl.

This product (1.4 g, 0.0031 mol) was dissolved in n-heptane (200 ml) and the solution was subjected to catalytic hydrogenation with Raney Ni (0.7 g) under atmospheric pressure at 60°C. The reaction was followed by gas chromatography and, after 12 hours, when the raw material had disappeared, the reduction was complete. Since the resulting material was a mixture of cis- and trans-isomers, it was recrystallized from toluene to isolate the trans-isomer, that is the desired product, 4-pentyl-4'-[trans-4-(trans-4-pentylcyclohexyl)cyclohexyl]-biphenyl. Yield: 0.5 g (0.001 mol). This product had a very broad liquid-crystalline temperature range, with a C—Sm point of room temperature or lower and a Sm—I point of 300°C or higher.

Example 7

Preparation of 4-ethyl-4'-[trans-4-(trans-4-pentylcylohexyl)cyclohexyl]biphenyl (a compound of the formula (V) wherein R = $C_5H_{11}$ and R' = $C_2H_5$)

A solution of 4-bromobiphenyl (34.5 g, 0.148 mol) dissolved in tetrahydrofuran (100 ml) was added dropwise to metallic Mg (3.6 g, 0.148 mol), and the reaction temperature was kept at 35°C under a nitrogen current. After 2 hours, the Mg had dissolved to form a uniform solution, to which was added dropwise a solution of 4-(trans-4-pentylcylohexyl)cylohexanone (29.6 g, 0.118 mol) in tetrahydrofuran (100 ml) with cooling to 30°C or below. The mixture was then refluxed for 2 hours and acidified with 3N hydrochloric acid (200 ml), followed by extraction with toluene (1 litre), washing with water and distilling off the solvent under reduced pressure, to obtain a residue which was 4-[4-(trans-4-pentylcylohexyl)cyclohexan-1-ol]biphenyl.

To this material was added potassium hydrogen sulphate (7 g) and the mixture was dehydrated at 200°C under a nitrogen current, followed by cooling, dissolving it in toluene (1 litre), filtering off the potassium hydrogen sulphate, washing the toluene layer with water, distilling off the solvent under reduced pressure and recrystallizing from toluene to obtain crystals of 4-[4-(trans-4-pentylcyclohexyl)cyclohexen-1-yl]biphenyl. This material (14.0 g, 0.0362 mol) was taken up in ethanol (300 ml) and reduced with Raney Ni (4.0 g) under 5 Kg/$cm^2$ of pressure at 80°C for 3 hours in an autoclave, followed by filtering off the catalyst, washing with toluene and recrystalizing, to obtain 4-[trans-4-(trans-4-pentylcyclohexyl)cyclohexyl]biphenyl.

This material (2.0 g, 0.0052 mol) was dissolved in $CS_2$ (100 ml), anhydrous $AlCl_3$ (1.0 g, 0.0077 mol) was suspended in the resulting solution, and then acetyl chloride (0.42 g, 0.0054 mol) was added dropwise while the temperature was kept at 0 to 10°C. After 1.5 hour, the temperature was gradually raised and the mixture was refluxed for 2 hours, followed by cooling, pouring the contents into 6N hydrochloric acid (500 ml), filtering the resulting solids and recrystallizing the solids from ethanol, to obtain 4-acetyl-4'-[trans-4-(trans-4-pentylcyclohexyl)cyclohexyl]biphenyl.

This material (1.7 g, 0.0039 mol) was dissolved in ethylene glycol (200 ml), and KOH (0.49 g) and hydrated hydrazine (0.52 g were added, followed by refluxing for 4 hours, distilled off ethylene glycol, extracting with toluene (200 ml), washing with 6N hydrocholoric acid, further washing with water till the washing water became neutral, distilling off toluene under reduced pressure and recrystallizing from fresh toluene, to obtain the desired product, 4-ethyl-4'-[trans-4-(trans-4-pentylcyclohexyl)cyclohexyl]biphenyl (0.2 g), which had a C-Sm point of 68.0°C, a Sm-N point of 290°C and a N—I point of 300°C or higher.

Examples 8 to 11

Compounds of the formula (V) having different substituents were prepared in a manner analogous to that of Example 6. Their phase transition points are shown in Table 2, together with those for the production of Examples 6 and 7.

TABLE 2

| Example | In formula (V) | | Amount of 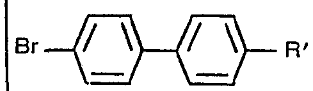 used (g) | Amount of 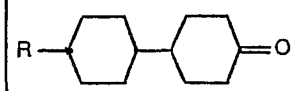 used (g) | Amount of 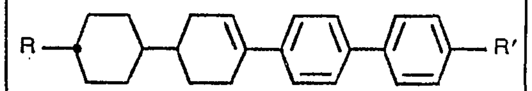 used (g) | Yield (g) (mol) | Phase transition point (°C) | |
|---|---|---|---|---|---|---|---|---|
| | R | R' | | | | | | |
| 8 | $C_3H_7$ | $C_2H_5$ | 12.8 | 8.0 | 5.4 (0.014 mol) | 0.9 (0.002 mol) | C—Sm<br>Sm—N<br>N—I | 71.4<br>279<br>300°C or higher |
| 9 | $C_4H_9$ | $C_2H_5$ | 12.8 | 9.5 | 4.4 (0.011 mol) | 1.1 (0.0027 mol) | C—Sm<br>Sm—I | 106.4<br>300°C or higher |
| 7 | $C_5H_{11}$ | $C_2H_5$ | 12.8 | 10.0 | 2.0 (0.0048 mol) | 0.2 (0.0005 mol) | C—Sm<br>Sm—N<br>N—I | 68.0<br>290<br>300°C or higher |
| 6 | $C_5H_{11}$ | $C_5H_{11}$ | 2.8 | 2.3 | 1.4 (0.0031 mol) | 0.3 (0.0007 mol) | C—Sm<br>Sm—I | room temp. or lower<br>300°C or higher |
| 10 | $C_5H_{11}$ | $OCH_3$ | 12.9 | 10.0 | 2.3 (0.0055 mol) | 0.4 (0.001 mol) | C—Sm<br>Sm—N<br>N—I | 87.2<br>260<br>300°C or higher |
| 11 | $C_5H_{11}$ | $OC_3H_7$ | 18.8 | 17.5 | 2.2 (0.0049 mol) | 0.7 (0.0016 mol) | C—Sm<br>Sm—I | 89.2<br>300°C or higher |

Example 12

Preparation of 4-ethyl-4'-(trans-4-ethylcyclohexyl)terphenyl (a compound of the formula (VI) wherein R = R = $C_2H_5$)

4-Ethyl-4'-[4'-(trans-4-ethylcyclohexyl)cyclohexen-1-yl]biphenyl (11 g, 0.030 mol) was dissolved in xylene (300 ml), chloranil (16 g, 0.065 mol) was added, and the reaction mixture was refluxed for 20 hours. After cooling, toluene (500 ml) was added, followed by washing once with 6N hydrochloric acid and three times with 2N NaOH, washing with saturated aqueous NaC1 solution till the washings became neutral, distilling off the solvent under reduced pressure, and recrystallizing from toluene, to obtain the desired product, 4-ethyl-4'-(trans-4-ethylcyclohexyl)terphenyl (yield: 5 g), having a C—Sm point of 147.0°C, a Sm-N point of 260°C and a N—I point of 300°C or higher.

Examples 13 to 17

Other compounds of the formula (VI) were prepared in a manner analogous to that of Example 12. Their properties are shown in Table 3, together with those for the product of Example 12.

TABLE 3

| Example | In formula (VI) | | Amount of 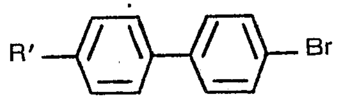 used (g) | Amount of 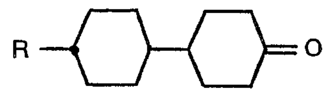 used (g) | Yield (g) | Phase transition point (°C) | | |
|---|---|---|---|---|---|---|---|---|
| | R | R′ | | | | C—Sm | Sm—N | N—I |
| 12 | $C_2H_5$ | $C_2H_5$ | 12.5 (0.049 mol) | 8.3 (0.040 mol) | 5.0 | 147.0 | 260 | 300< |
| 13 | $C_2H_5$ | $C_5H_{11}$ | 14.5 (0.049 mol) | 8.3 (0.040 mol) | 1.0 | room temp. or lower | 281 | 300< |
| 14 | $C_4H_9$ | H | 11.4 (0.049 mol) | 9.5 (0.040 mol) | 4.4 | 195.7 (C—N) | — | 235 |
| 15 | $C_5H_{11}$ | H | 11.4 (0.049 mol) | 10.0 (0.040 mol) | 2.7 | 134.7 | 261 | 284 |
| 16 | $C_5H_{11}$ | $C_2H_5$ | 12.5 (0.049 mol) | 10.0 (0.040 mol) | 4.0 | 91.1 | 294 | 300< |
| 17 | $C_5H_{11}$ | $C_5H_{11}$ | 14.5 (0.049 mol) | 10.0 (0.040 mol) | 3.0 | room temp. or lower | 300< | — |

Example 18

Preparation of 4-methyl-{4-[trans-4-(trans-4-ethylcyclohexyl)cyclohexyl]cyclohexen-1-ly}-benzene (a compound of the formula (IV) wherein R = ethyl and R′ = methyl)

Sliced Mg (1.2 g, 0.0049 mol) was placed in a three-neck flask, and a solution (30 ml) of 4-bromoanisole (9.2 g, 0.049 mol) dissolved in tetrahydrofuran was slowly added dropwise to the Mg under a nitrogen current with stiring while the reaction temperature was kept at 30 to 35°C. The Mg dissolved in 3 hours, as the reaction proceeded, to form a uniform solution of 4-methoxyphenylmagnesium bromide; and to this a solution (50 ml) of 4-trans-4-ethylcyclohexyl)cyclohexanone (10.2 g, 0.049 mol) dissolved in tetrahydrofuran was added dropwise as rapidly as possible while the reaction temperature was kept at 5 to 10°C. After the addition, the temperature was raised to 35°C, followed by stirring for 30 minutes. The reaction mixture was acidified with hydrochloric acid (50 ml) and extracted three times with toluene (200 ml) in a separating funnel. The combined toluene layers were washed with water till the washings became neutral and the toluene was distilled off under reduced pressure, to obtain 4-methoxy-[1-hydroxy-4-(trans-4-ethylcyclohexyl)cyclohexyl]benzene as an oily residue.

Potassium hydrogen sulphate (6 g) was added to this oil, followed dehydrating it at 160°C for 2 hours under a nitrogen current, cooling, adding toluene (200 ml), filtering off the potassium hydrogen sulphate, washing the toluene layer with water till the washings became neutral, distilling off the toluene under reduced pressure and recrystallizing the remaining oily substance from ethanol, to obtain 4-methoxy-[4-(trans-4-ethylcyclohexyl)cyclohexanen-1-yl]benzene. This material (70 g) was dissolved in ethanol (120 ml) and subjected to catalytic hydrogenation with Raney Ni (1.0 g) under atmospheric pressure at 50°C, using 500 ml of hydrogen, followed by filtering off the catalyst and recrystallizing the product. Since the resulting material was a mixture of cis- and trans-isomers, it was repeatedly recrystallized from ethanol to isolate the trans-isomer.

The resulting 4-methoxy-[trans-4-(trans-4-ethylcyclohexyl)cyclohexyl]benzene (80 g) was dissolved in acetic acid (450 ml), 47% hydrobromic acid (450 ml) was added, and the mixture was refluxed for 30 hours. After this, the reaction mixture was cooled, and the crystals which were deposited were filtered off and recrystallized from ethanol to obtain 4-hydroxy-[trans-4-(trans-4-ethylcyclohexyl)cyclohexyl]benzene.

This material (20 g) was placed in an autoclave with ethanol (400 ml) and subjected to catalytic reduction with Ru/C (3 g) under 50 Kg/cm$^2$ at 180°C, followed by filtering off the catalyst and distilling off the solvent under reduced pressure, to obtain 4-[trans-4-(trans-4-ethylcyclohexyl)cyclohexyl]cyclohexanol. To this material was added acetone (8 litres), and a solution (50 ml) obtained by adding water to anhydrous chromic acid (14.5 g) and concentrated sulphuric acid (23.6 g) was added dropwise over 2 hours while the temperature was kept at 0°C to 3°C.

After completion of the reaction, the excess of the oxidising agent was decomposed by adding isopropyl alcohol. The reaction mixture was neutralized by adding sodium hydrogen carbonate, followed by filtering off the resulting precipitate, washing it with acetone, combining the filtrate and the washings, distilling the acetone under reduced pressure, extracting three times with toluene (500 ml), drying the combined toluene layers with sodium sulphate, distilling off the toluene under reduced pressure, and recrystallizing the residues from fresh toluene (50 ml), to obtain 4-[trans-4-(trans-4-ethylcyclohexyl)cyclohexyl]cyclohexanone (XII), which was then dried by heating *in vacuo*.

This material (11.6 g) was dissolved in tetrahydrofuran (100 ml) and the solution was rapidly added dropwise to a solution of 4-methylbenzenemagensium bromide obtained by reacting Mg pieces (1.2 g, with p-bromotoluene (8.38 g). After refluxing for 3 hours, the reaction liquid was cooled and acidified with 3N hydrochloric acid, followed by extraction with toluene (300 ml), repeatedly washing with water till the washings became neutral, distilling off the solvent under reduced pressure, adding potassium hydrogen sulphate (4 g) to the remaining oily substance, dehydrating at 160°C for 2 hours in a nitrogen atmosphere, cooling, filtering off the potassium hydrogen sulphate, adding toluene (300 ml), washing with water, distilling off the solvent under reduced pressure, and recrystallizing from fresh toluene (70 ml) to obtain the desired product, 4-methyl-4-[trans-4-(trans-4-ethylcyclohexyl)cyclohexyl]cyclohexen-1-yl benzene (yield: 4.6 g) having a C—Sm point of 59.4°C, a Sm—N point of 234°C and a N—I point of 284°C.

Example 19

Preparation of 4-methyl-trans-4-[trans-4-trans-4-ethylcyclohexyl)cyclohexyl]cyclohexyl benzene (a compound of the formula (III) wherein R = ethyl and R′ = methyl)

4-Methyl-4-[trans-4-trans-4-ethylcyclohexyl)cyclohexyl(cyclohexyl]-cyclohexen-1-yl benzene (3.2 g) was dissolved in toluene (300 ml) and subjected to catalytic hydrogenation with Raney Ni (1.5 g) under atmospheric pressure at room temperature. When the reaction was finished, as indicated by monitoring the disappearence of the starting material, by means of gas chromatography, the solvent was distilled off and the residue recrystallized from toluene, to obtain 4-methyl-trans-4-[trans-4-(trans-4-ethylcyclohexyl)cyclohexyl]cyclohexyl benzene (yield: 0.8 g) having a C—Sm point of 71.0°C, a Sm—N point of 254°C and a N—I point of 293°C.

Example 20

Preparation of 4,4′-bis(4-propylphenyl)bicyclohexan-3,3′-diene (a compound of the formula (VIII) wherein R = propyl)

Mg pieces (3.7 g, 0.15 mol) were reacted with 4-propyl-bromobenzene (30 g, 0.15 mol) in tetrahydrofuran at about 50°C to obtain 4-propylbenzenemagnesium bromide, and a solution of crystals of 4,4′-bicyclohexyldione (11.7 g, 0.06 mol) dissolved in tetrahydrofuran (200 ml) was added to obtain 4,4′-bis(4-propylhenyl)bicyclohexan-4,4′-diol. To this material was added potassium hydrogen sulphate (6 g), followed by dehydration at 200°C for 2 hours under a nitrogen current and recrystallization from toluene, to obtain 4-4′-bis(4-propylhenyl)bicyclohexan-3,3′-diene (yield: 4.7 g) having a C—Sm point of 109.7°C, a Sm—N point of 243°C and a N—I point of 259°C.

Examples 21 and 22

Compounds having different substituents were prepared in a manner analogous to that of Example 20. Their physical properties are shown in Table 4 together with those of the product of Example 20. The chemical structures of the compounds were confirmed by NMR.

TABLE 4

| Example | R in formula (VIII) | Yield (g) | Yield (%)* | Phase transition point (°C) | | |
|---|---|---|---|---|---|---|
| | | | | C—N(Sm) point | Sm—N point | N—I point |
| 20 | $C_3H_7$ | 4.7 | 20 | 109.7 | 243 | 259 |
| 21 | $CH_3$ | 47 | 27 | 198.7 ~ 199.0 | — | 263 |
| 22 | $C_3H_7O$ | 38 | 17 | 151 | 251 ~ 253 | 283 |

* Values based on bicyclohexanedione.

Example 23

Preparation of trans,trans-4-4′-bis(4-propylphenyl)bicyclohexane (a compound of the formula (VII) wherein R = propyl)

(4-Propylphenyl)bicyclohexan-3,3′-diene (4.7 g) was dissolved in toluene (200 ml) and subjected to catalytic hydrogenation with Raney Ni (2 g) under 3 Kg/cm$^2$ at 80°C in an autoclave. The reaction was stopped on the disappearance of the starting material, as observed by gas chromatography, followed by filtering off the catalyst and recrystallizing the product to obtain trans,trans-4-4′-bis(4-propylphenyl)bicyclohexane (VII) (yield: 1.3 g) having a C—Sm point of 104.7°C, a Sm—N point of 246°C and a N—I point of 290°C.

Examples 24 and 25

Compounds having different substituents were prepared in an analogous manner to that of Example 23. Their physical properties are shown in Table 5, together with those of the product of Example 23. The chemical structures of the compounds were confirmed by NMR.

TABLE 5

| Example | R in formula (VII) | Phase transition point (°C) | | |
|---|---|---|---|---|
| | | C—N(Sm) point | Sm—N point | N—I point |
| 23 | $C_3H_7$ | 104.0 | 246 | 290 |
| 24 | $CH_3$ | 190.4 ~ 190.7 | — | 269 |
| 25 | $C_3H_7O$ | 158.1 ~ 159.0 | 245 | 300 or higher |

Example 26

Preparation of 1,4-bis[4-(trans-4-propylcyclohexyl)cyclohexen-1-yl]benzene (a compound of the formula (X) wherein R = propyl)

A solution of p-dibromobenzene (5.6 g, 0.024 mol) dissolved in tetrahydrofuran (200 ml) was added dropwise to sliced Mg (1.2 g, 0.049 mol) under a nitrogen current so as to give a gentle reflux. After 7 hours, the reaction finished to yield the magnesium bromide, to which a solution of 4-(trans-4-propylcyclohexyl)cyclohexanone (10.9 g, 0.049 mol) dissolved in tetrahydrofuran (100 ml) was rapidly added dropwise while the temperature was kept at 30°C or lower. The resulting mixture was refluxed for 1.5 hour and then acidified with 3N hydrochloric acid. The reaction liquid was extracted with toluene (100 ml × 3), and the combined toluene layers were washed with water and the solvent was distilled off under reduced pressure.

The remaining oily substance was 1,4-bis[4-(trans-4-propylcyclohexyl)cyclohexan-1-ol]benzene, to which potassium hydrogen sulphate (2.5 g) was added, followed by dehydrating at 200°C for 2 hours under a nitrogen current, cooling, adding toluene (300 ml), filtering off the potassium hydrogen sulphate, washing the toluene layer with water, distilling off the solvent under reduced pressure and recrystallizing the residue from tuluene, to obtain the desired product, 1,4-bis[4-(trans-4-propylcyclohexyl)cyclohexen-1-yl]benzene (yield: 0.9 g) having a C—Sm point of 98.5°C, a Sm—I point of 300°C or higher.

Example 27

Example 26 was repeated, except that the 4-(trans-4-propylcyclohexyl)cyclohexanone in Example 26 was replaced by 4-(trans-4-pentylcyclohexyl)cyclohexanone, to prepare 1,4-bis[4-(trans-4-pentylcyclohexyl)cyclohexan-1-yl]benzene (C—Sm point: 47.7°C, Sm—I point: 300°C or higher).

Example 28

Preparation of 1,4-bis[trans-4-(trans-4-propylcyclohexyl)cyclohexyl]benzene (a compound of the formula (IX) wherein R = propyl)

Raney Ni catalyst (0.5 g) was added to a solution of 1,4-bis[4-(trans-4-propylcyclohexyl)cyclohexen-1-yl]benzene (0.9 g) dissolved in toluene (70 ml), and catalytic hydrogenation was carried out at atmospheric temperature and pressure. The reaction was followed by gas chromatography and stopped when the starting material disappeared. Since the product was a mixture of cis- and trans-isomers, it was recrystallized from toluene to obtain the desired product, 1,4-bis[trans-4-(trans-4-propylcyclohexyl)cyclohexyl]benzene (yield: 0.3 g, C—Sm point: room temperature or lower, Sm—I point: 300°C or higher).

Example 29

Example 26 was repeated, except that the 4-(trans-4-propylcyclohexyl)cyclohexanone was replaced by 4-(trans-4-pentylcyclohexyl)cyclohexanone, to prepare 1,4-bis[trans-4-(trans-4-propylcyclohexyl)cyclohexyl]-benzene (C—Sm point: 47.7°C, Sm—I point: 300°C or higher).

Example 30

A liquid-crystalline composition (A) consisting of:- trans-4-propyl-(4′-cyanophenyl)cyclohexane 28%, trans-4-pentyl-(4′-cyanophenyl)cyclohexane 43%, and trans-4-heptyl-(4′-cyanophenyl)cyclohexane 29% has a N—I point of 52°C, a dielectric anisotropy value $\Delta\varepsilon$ of +10.5, a threshold voltage of 1.53 V and a saturation voltage of 2.12 V relative to a TN cell having the composition sealed therein, a viscosity at 20°C of 23 cp and an optical anisotropy value $\Delta n$ of 0.12.

When 1-(trans-4-ethylcyclohexyl)-4-[trans-4-(trans-4-propylcyclohexyl)cyclohexyl]benzene (5 parts) obtained in Example 3 of the present invention was added to the above liquid crystal mixture (95 parts), the resulting liquid-crystalline composition had a raised N—I point of 60.5°C, a $\Delta\varepsilon$ of +10.6, a threshold voltage of 1.70 V, a saturation voltage of 2.5 V relative to a TN cell having this composition sealed therein and a viscosity at 20°C of 30.3.cp.

Thus, it is seen that addition of the compound of the present invention in a small amount is effective for broadening the nematic temperature range.

Example 31

When 4-pentyl-4′-[trans-4-(trans-4-pentylcyclohexyl)cyclohexyl]biphenyl of Example 6 of the present invention (2 parts) was added to the above liquid-crystalline composition (A) (98 parts), the resulting liquid-crystalline composition had a raised N—I point of 56.2°C, a $\Delta\varepsilon$ of +11.0, a threshold voltage of 1.55 V, a saturation voltage of 2.15 V relative to a TN cell having this composition sealed therein and a viscosity at 20°C of 24.9 cp.

Thus, it is seen that addition of the compound of the present invention in a small amount is effective for preparing a composition having a low viscosity and a broad nematic temperature range.

Example 32

When 4-ethyl-4′-(trans-4-pentylcyclohexyl)terphenyl of Example 16 of the present invention (5 parts) was added to the above liquid-crystalline composition (A) (95 parts), the resulting liquid-crystalline

composition had a raised N—I point of 61.5°C, a threshold voltage of 1.62 V, a saturation voltage of 2.44 V, a viscosity at 20°C of 24.8 and a raised Δn of 0.126. Thus, it is seen that addition of the compound of the present invention in a small amount is effective for preparing a composition having a low viscosity and a broad nematic temperature range.

Example 33

When 4-methyl-{4-[trans-4-(trans-4-ethylcyclohexyl)-cyclohexyl]cyclohexen-1-yl}benzene of Example 18 of the present invention (5 parts) was added to the above liquid-crystalline composition (A) (95 parts), the resulting liquid-crystalline composition had a raised N—I point of 61.9°C, a threshold voltage of 1.6 V, a saturation voltage of 2.2 V, a viscosity at 20°C of 25 cp. Thus, it was possible to obtain a liquid-crystalline composition having a low viscosity and a broad nematic temperature range.

Example 34

When 4-methyl-{[trans-4-[trans-4-(trans-4-ethylcyclohexyl)-cyclohexyl]cyclohexyl}benzene of Example 19 of the present invention (5 parts) was added to the above liquid-crystalline composition (A) (95 parts), the resulting liquid-crystalline composition had a raised N—I point of 61.5°C; its viscosity at 20°C of 25 cp, that is, did not rise greatly; the threshold voltage and saturation voltage were 1.5 V and 2.1 V, respectively; thus, it was possible to broaden the nematic temperature range without raising these voltages.

EXAMPLE 35

When 4,4′-bis(4-propylphenyl)bicyclohexan-3,3′-diene of Example 20 of the present invention (2.5 parts) was added to the above liquid-crystalline composition (A) (97.5 parts), the resulting liquid-crystalline composition had a raised N—I point of 56.4°C, a viscosity at 20°C of 26.1 cp, a threshold voltage of 1.58 V, and a saturation voltage of 2.15 V. Thus, addition of the compound of the present invention in a small amount made it possible to broaden nematic temperature range without greatly raising the viscosity.

Example 36

When trans,trans-4-4′-bis(4-propylphenyl)bicyclohexane of Example 23 of the present invention (5 parts) was added to the above liquid-crystalline composition (A) (95 parts), the resulting liquid-crystalline composition had a raised N—I point of 59.1°C; a viscosity at 20°C of 24.9 cp, a threshold voltage of 1.64 V, and a saturation voltage of 2.27 V, Thus, addition of the compound of the present invention in a small amount made it possible to broaden the nematic temperature range without greatly raising the viscosity.

Example 37

A liquid-crystalline composition consisting of:- trans-4-propyl-(4′-cyanophenyl)cyclohexane 25.5%, trans-4-pentyl-(4′-cyanophenyl)cyclohexane 34.0% trans-4-heptyl-(4′-cyanophenyl)cyclohexane 25.5% and trans-4-pentyl-(4′-cyanobiphenyl)cyclohexane 15.0% has a nematic liquid-crystalline temperature range of −6°C to +72.3°C, a viscosity at 20°C of 27.6 cp, a dielectric anisotropy value Δξ of 11.5, and a TN cell having the composition sealed therein has a threshold voltage of 1.69 V and a saturation voltage of 2.45 V.

When 1,4-bis[4-(trans-4-propylcyclohexyl)cyclohexen-1-yl]benzene of Example 26 of the present invention (one part) was added to the above liquid-crystalline composition (99 parts), the resulting liquid-crystalline composition had a broadened nematic temperature range of −6°C to +75.0°C, a viscosity at 20°C of 31 cp, a dielectric anisotropy of +10.9, and a TN cell having this composition sealed therein had a threshold voltage of 1.71 V and a saturation voltage of 2.4 V.

Example 38

When 1,4-bis[trans-4-(trans-4-pentylcyclohexyl)cyclohexyl]benzene of Example 29 of the present invention (2 parts) was added to the above composition (A) (98 parts), the resulting liquid-crystalline composition had raised N—I point of 56.7°C, a Δξ of +10.8, a threshold voltage of 1.54 V and a saturation voltage of 2.1 V relative to a TN cell having this composition sealed therein, and a viscosity at 20°C of 26 cp. Thus, addition of the compound of the present invention in a small amount is effective for broadening the nematic temperature range.

**Claims:**

1. Compounds of the following general formulae (II) to (X):-

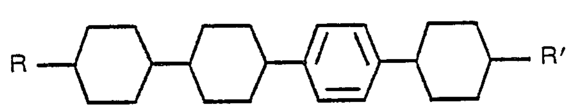

(II)

wherein each of R and R′ represents a hydrogen atom or an alkyl group of 1 to 10 carbon atoms;

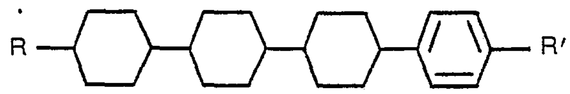

(III)

wherein each of R and R′ represents a hydrogen atom, or an alkyl or alkoxy group, each of 1 to 10 carbon atoms;

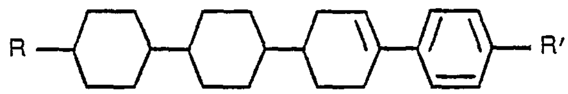

(IV)

wherein each of R and R′ represents a hydrogen atom, or an alkyl or alkoxy group, each of 1 to 10 carbon atoms;

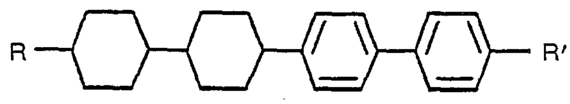

(V)

wherein each of R and R′ represents a hydrogen atom, or an alkyl or alkoxy group, each of 1 to 10 carbon atoms;

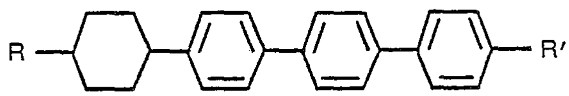

(VI)

wherein each of R and R′ represents a hydrogen atom, or an alkyl or alkoxy group, each of 1 to 10 carbon atoms;

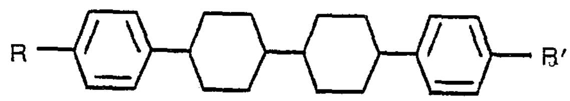

(VII)

wherein R represents a hydrogen atom, or an alkyl or alkoxy group, each of 1 to 10 carbon atoms;

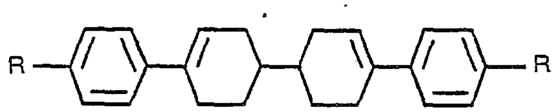

(VIII)

wherein R represents a hydrogen atom, or an alkyl or alkoxy group, each of 1 to 10 carbon atoms;

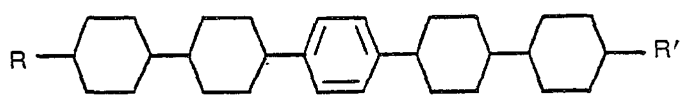

(IX)

wherein each of R and R′ represents a hydrogen atom, or an alkyl group of 1 to 10 carbon atoms;

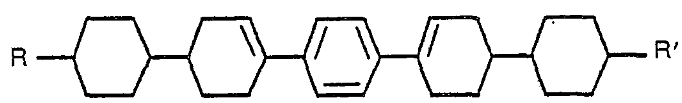

(X)

wherein each of R and R′ represents a hydrogen atom, or an alkyl group of 1 to 10 carbon atoms;

2. 1-(Trans-4-alkylcyclohexyl)-4-[trans-4-(trans-4-alkylcyclohexyl)cyclohexyl]benzenes of the formula:

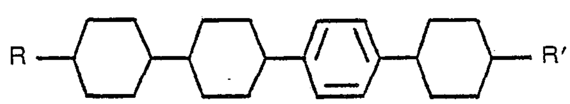

wherein each of R and R′ represents a hydrogen atom or an alkyl group having from 1 to 10 carbon atoms.

3. 4-Substituted-trans-4-[trans-4-(trans-4-alkylcyclohexyl)cyclohexyl]benzenes of the formula:

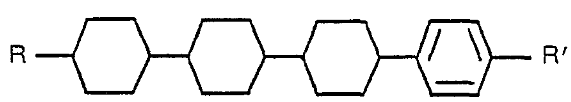

wherein each of R and R′ represents a hydrogen atom, or an alkyl or alkoxy group having from 1 to 10 carbon atoms.

4. 4-Substituted-{4-[trans-4-(trans-4-alkylcyclohexyl)cyclohexyl]cyclohexen-1-yl]benzenes of the formula:

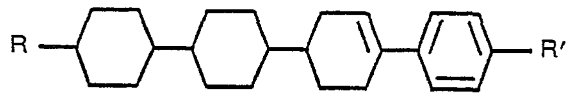

wherein each of R and R′ represents a hydrogen atom, or an alkyl or alkoxy group having from 1 to 10 carbon atoms.

5. 4-Substituted-4-[trans-4-(trans-4-substituted-cyclohexyl)cyclohexyl]biphenyls of the formula:

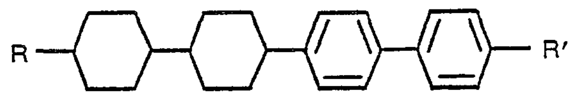

wherein each of R and R′ represents a hydrogen atom, or an alkyl or alkoxy group having from 1 to 10 carbon atoms.

6. 4-Substituted-4′-(trans-4-alkylcyclohexyl)terphenyls of the formula:

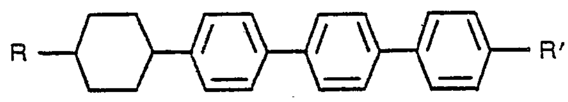

wherein each of R and R′ represents a hydrogen atom, or an alkyl or alkoxy group having from 1 to 10 carbon atoms.

7. Trans,trans-4,4′-bis(4-substituted-phenyl)bicyclohexanes of the formula:

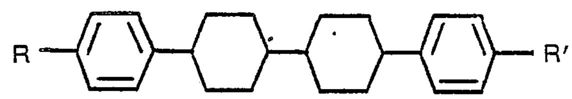

wherein R and R′ represents a hydrogen atom, or an alkyl or alkoxy group having from 1 to 10 carbon atoms.

8. 4,4′-Bis(4-substituted-phenyl)bicyclohexan-3,3′-dienes of the formula:

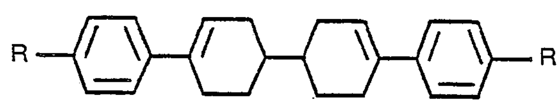

wherein R represents a hydrogen atom, or an alkyl or alkoxy group having from 1 to 10 carbon atoms.

9. 1,4-Bis[trans-4-trans-4-alkylcyclohexyl)cyclohexyl]-benzenes of the formula:

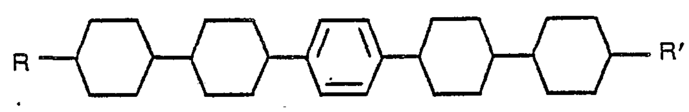

wherein each of R and R′ represents a hydrogen atom or an alkyl group having from 1 to 10 carbon atoms.

10. 1,4-Bis[4-trans-4-alkylcyclohexyl)cyclohexen-1-yl]-benzenes of the formula:

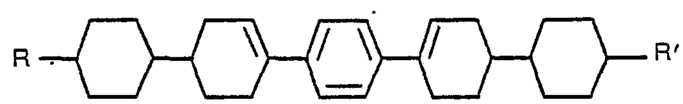

wherein each of R and R′ represents a hydrogen atom or an alkyl group having from 1 to 10 carbon atoms.

11. A liquid-crystalline composition containing at least one compound according to any of claims 1 to 10.

**Patentansprüche**

1. Verbindungen der nachfolgenden allgemeinen Formel II bis X:

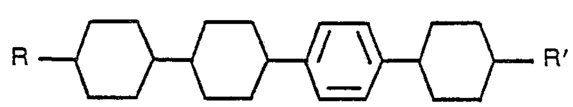

(II)

worin R und R′ je ein Wasserstoffatom oder eine Alkylgruppe mit 1 bis 10 Kohlenstoffatomen bedeuten;

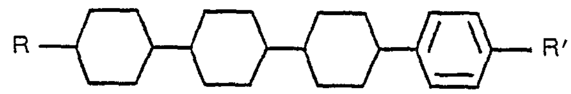

(III)

worin R und R′ je ein Wasserstoffatom oder eine Alkylgruppe mit 1 bis 10 Kohlenstoffatomen oder eine Alkoxygruppe mit 1 bis 10 Kohlenstoffatomen bedeuten;

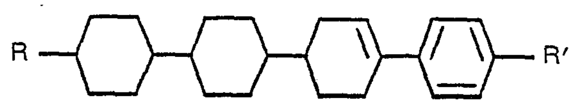

(IV)

worin R und R′ je ein Wasserstoffatom oder eine Alkylgruppe mit 1 bis 10 Kohlenstoffatomen oder eine Alkoxygruppe mit 1 bis 10 Kohlenstoffatomen bedeuten;

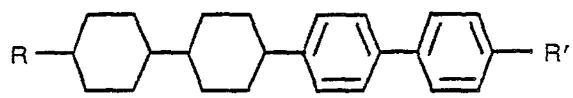

(V)

worin R und R′ je ein Wasserstoffatom oder eine Alkylgruppe mit 1 bis 10 Kohlenstoffatomen oder eine Alkoxygruppe mit 1 bis 10 Kohlenstoffatomen bedeuten;

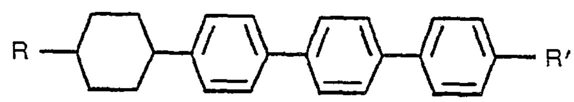

(VI)

worin R und R′ je ein Wasserstoffatom oder eine Alkylgruppe mit 1 bis 10 Kohlenstoffatomen oder eine Alkoxygruppe mit 1 bis 10 Kohlenstoffatomen bedeuten;

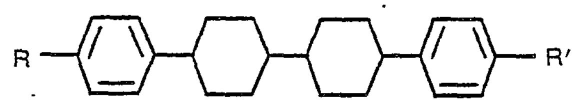

(VII)

worin R ein Wasserstoffatom oder eine Alkylgruppe mit 1 bis 10 Kohlenstoffatomen oder eine Alkoxygruppe mit 1 bis 10 Kohlenstoffatomen bedeuten;

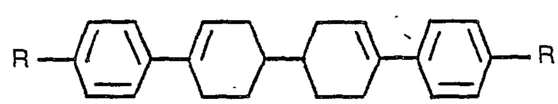

(VIII)

worin R ein Wasserstoffatom oder eine Alkylgruppe mit 1 bis 10 Kohlenstoffatomen oder eine Alkoxygruppe mit 1 bis 10 Kohlenstoffatomen bedeuten;

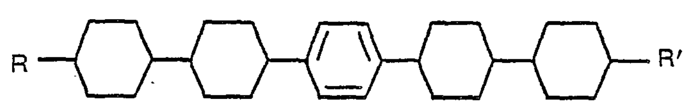

(IX)

worin R und R′ je ein Wasserstoffatom oder eine Alkylgruppe mit 1 bis 10 Kohlenstoffatomen bedeuten;

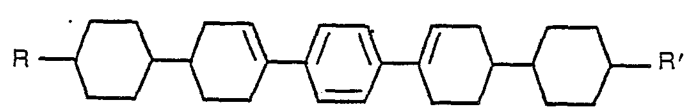

(X)

worin R und R′ je ein Wasserstoffatom oder eine Alkylgruppe mit 1 bis 10 Kohlenstoffatomen bedeuten.

2. 1-(Trans-4-alkylcyclohexyl)-4-[trans-4-(trans-4-alkylcyclohexyl)cyclohexyl]benzene der Formel:

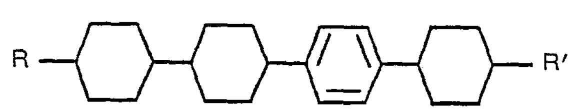

worin R und R′ ein Wasserstoffatom oder eine Alkylgruppe mit 1 bis 10 Kohlenstoffatomen bedeuten.

3. In 4-Stellung substituierte Trans-4-[trans-4-(trans-4-alkylcyclohexyl)cyclohexyl]benzene der Formel:

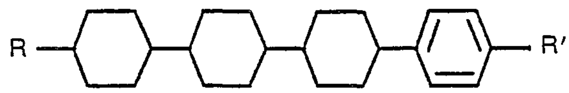

worin R und R' ein Wasserstoffatom oder eine Alkylgruppe mit 1 bis 10 Kohlenstoffatomen oder eine Alkoxygruppe mit 1 bis 10 Kohlenstoffatomen bedeuten;

4. In 4-Stellung substituierte {4-[Trans-4-(trans-4-alkylcyclohexyl)cyclohexyl]cyclohexen-1-yl}benzene der Formel:

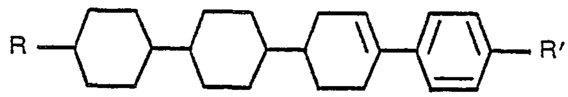

worin R und R' je ein Wasserstoffatom oder eine Alkylgruppe mit 1 bis 10 Kohlenstoffatomen oder eine Alkoxygruppe mit 1 bis 10 Kohlenstoffatomen bedeuten;

5. In 4-Stellung substituierte 4-[Trans-4-(trans-4-substituierte-cyclohexyl)cyclohexyl]biphenyle der Formel:

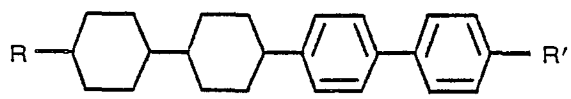

worin R und R' je ein Wasserstoffatom oder eine Alkylgruppe mit 1 bis 10 Kohlenstoffatomen oder eine Alkoxygruppe mit 1 bis 10 Kohlenstoffatomen bedeuten.

6. In 4-Stellung substituierte 4'-(Trans-4-alkylcyclohexyl)terphenyle der Formel:

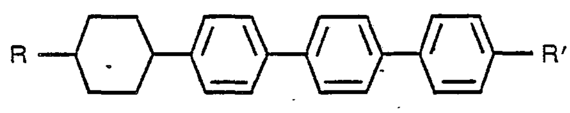

worin R und R' je ein Wasserstoffatom oder eine Alkylgruppe mit 1 bis 10 Kohlenstoffatomen oder eine Alkoxygruppe mit 1 bis 10 Kohlenstoffatomen bedeuten.

7. Trans,trans-4,4'-bis(4-substituierte-phenyl)bicyclohexane der Formel:

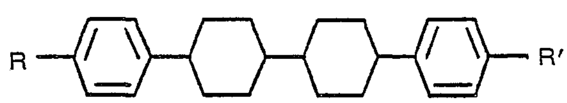

worin R und R' je ein Wasserstoffatom oder eine Alkylgruppe mit 1 bis 10 Kohlenstoffatomen oder eine Alkoxygruppe mit 1 bis 10 Kohlenstoffatomen bedeuten.

8. 4,4'-Bis(4-substituierte-phenyl)bicyclohexan-3,3'-diene der Formel:

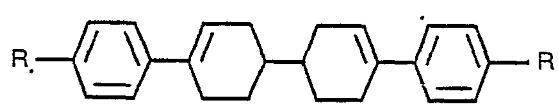

worin R ein Wasserstoffatom oder eine Alkylgruppe mit 1 bis 10 Kohlenstoffatomen oder eine Alkoxygruppe mit 1 bis 10 Kohlenstoffatomen bedeuten.

9. 1,4'-Bis[trans-4-(trans-4-alkylcyclohexyl)cyclohexyl]benzene der Formel:

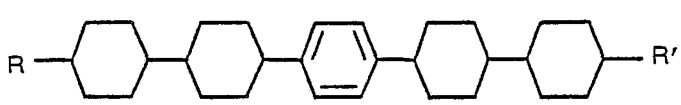

worin R und R' je ein Wasserstoffatom oder eine Alkylgruppe mit 1 bis 10 Kohlenstoffatomen bedeuten.

10. 1,4'-Bis[4-(trans-4-alkylcyclohexyl)cyclohexen-1-yl]-benzene der Formel:

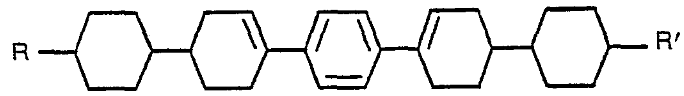

worin R und R' je ein Wasserstoffatom oder eine Alkylgruppe mit 1 bis 10 Kohlenstoffatomen bedeuten.

11. Flüssigkristallzubereitung enthalten mindestens eine Verbindung nach einem der Ansprüche 1 bis 10.

**Revendications**

1. Composés ayant les formules générales suivantes (II) à (X):

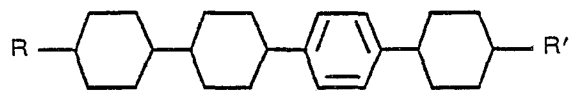

(II)

dans laquelle R et R' représentent chacun un atome d'hydrogène ou un groupement alkyle de l à 10 atomes de carbone;

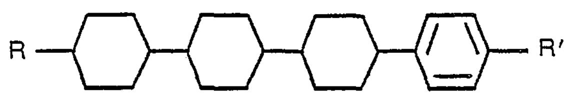

(III)

dans laquelle R et R' représentent chacun un atome d'hydrogène ou un groupement alkyle ou alcoxy, chacun ayant de l à 10 atomes de carbone;

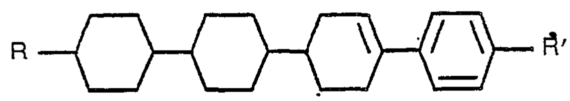

(IV)

dans laquelle R et R' représentent chacun un atome d'hydrogène ou un groupement alkyle ou alcoxy, chacun ayant de l à 10 atomes de carbone;

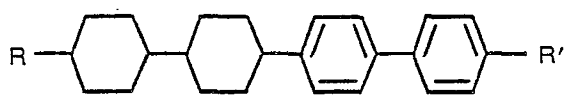

(V)

dans laquelle R et R' représentent chacun un atome d'hydrogène ou un groupement alkyle ou alcoxy, chacun ayant de l à 10 atomes de carbone;

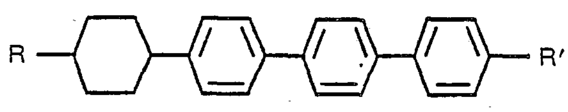

(VI)

dans laquelle R et R' représentent chacun un atome d'hydrogène ou un groupement alkyle ou alcoxy, chacun ayant de l à 10 atomes de carbone;

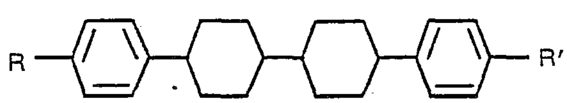

(VII)

dans laquelle R représente un atome d'hydrogène ou un groupement alkyle ou alcoxy, chacun ayant de l à 10 atomes de carbone;

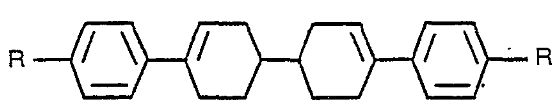

(VIII)

dans laquelle R représente un atome d'hydrogène ou un groupement alkyle ou alcoxy, chacun ayant de l à 10 atomes de carbone;

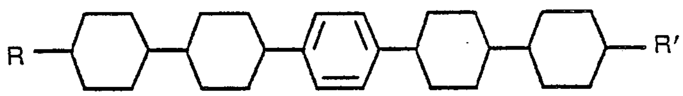

(IX)

dans laquelle R et R′ représentent un atome d'hydrogène ou un groupement alkyle de I à 10 atomes de carbone;

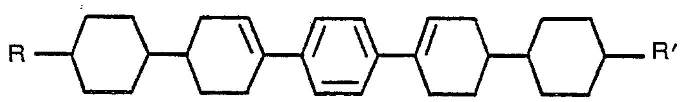

(X)

dans laquelle R et R′ représentent chacun un atome d'hydrogène ou un groupement alkyle de I à 10 atomes de carbone.

2. 1-(Trans-4-alkylcyclohexyl)-4-[trans-4-(trans-4-alkylcyclohexyl)cyclohexyl]benzènes de formule

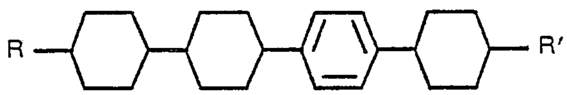

dans laquelle R et R′ représentent chacun un atome d'hydrogène ou un groupement alkyle de I à 10 atomes de carbone.

3. Trans-4-[trans-4-(trans-4-alkylcyclohexyl)cyclohexyl]cyclohexyl]-cyclohexylbenzènes de formule

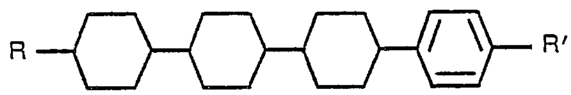

dans laquelle R et R′ représentent chacun un atome d'hydrogène ou un groupement alkyle ou alcoxy ayant de I à 10 atomes de carbone.

4. {4-[Trans-4-(trans-4-alkylcyclohexyl)cyclohexyl]cyclohexènyl-1}benzènes de formule

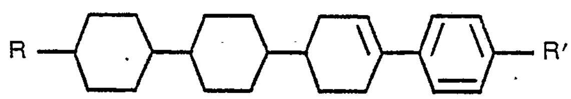

dans laquelle R et R′ représentent chacun un atome d'hydrogène ou un groupement alkyle ou alcoxy ayant de I à 10 atomes de carbone.

5. 4-[Trans-4-(trans-cyclohexyl-4-substitué)cyclohexyl]-biphényles 4-substitués de formule

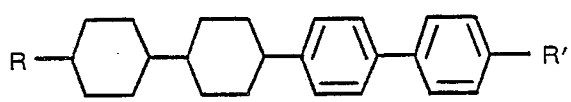

dans laquelle R et R′ représentent chacun un atome d'hydrogène ou un groupement alkyle ou alcoxy ayant de I à 10 atomes de carbone.

6. 4′-(Trans-4-alkylcyclohexyl)terphényles 4-substitués de formule

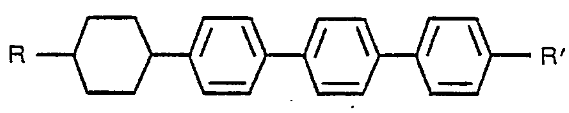

dans laquelle R et R′ représentent chacun un atome d'hydrogène ou un groupement alkyle ou alcoxy ayant de I à 10 atomes de carbone.

7. Trans,trans-4,4-bis(phényl 4-substitué) bicyclohexanes de formule

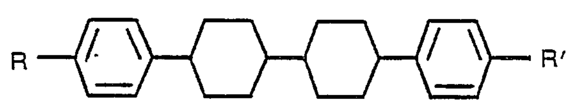

dans laquelle R et R′ représentent chacun un atome d'hydrogène ou un groupement alkyle ou alcoxy ayant de I à 10 atomes de carbone.

8. 4,4'-Bis(phényl 4-substitué) bicyclohexane-3,3-diènes de formule

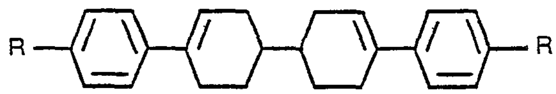

dans laquelle R représente un atome d'hydrogène ou un groupement alkyle ou alcoxy ayant de I à 10 atomes de carbone.

9. 1,4-Bis[trans-4-(trans-4-alkylcyclohexyl)-cyclohexyl]-benzènes de formule

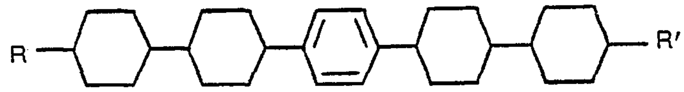

dans laquelle R et R' représentent chacun un atome d'hydrogène ou un groupement alkyle ayant de I à 10 atomes de carbone.

10. 1,4-Bis[4-(trans-4-alkylcyclohexyl)-cyclohexényl-1]-benzènes de formule

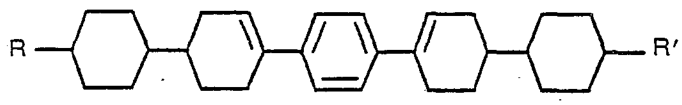

dans laquelle R et R' représentent un atome d'hydrogène ou un groupement alkyle ayant de I à 10 atomes de carbone.

11. Composition de cristal liquide contenant au moins un composé selon l'une quelconque des revendications 1 à 10..